# EUROPÄISCHE PATENTSCHRIFT

(11) **EP 2 688 671 B1**
(45) Veröffentlichungstag und Bekanntmachung des Hinweises auf die Patenterteilung: **11.10.2017**
(21) Anmeldenummer: 12711624.2
(22) Anmeldetag: 23.03.2012
(51) Int. Cl.: B01L 3/00, F16K 99/00

(54) **VORRICHTUNG ZUM HERSTELLEN EINER FLUIDISCHEN VERBINDUNG ZWISCHEN KAVITÄTEN**
DEVICE FOR PRODUCING A FLUIDIC CONNECTION BETWEEN CAVITIES
DISPOSITIF DE FABRICATION D'UNE LIAISON FLUIDIQUE ENTRE CAVITÉS

(30) Priorität: 24.03.2011 EP 11002439
(43) Veröffentlichungstag der Anmeldung: 29.01.2014
(73) Patentinhaber: Boehringer Ingelheim Microparts GmbH, 44227 Dortmund (DE)
(72) Erfinder: LANGE, Berthold, 55216 Ingelheim Am Rhein (DE); RODENFELS, Tobias, 55216 Ingelheim Am Rhein (DE); STOETERS, Wolfgang, 55216 Ingelheim Am Rhein (DE); VOIGT, Markus, 55216 Ingelheim Am Rhein (DE)
(74) Vertreter: Häckel, Stefan
(86) Internationale Anmeldenummer: PCT/EP2012/055263
(87) Internationale Veröffentlichungsnummer: WO 2012/127049

(56) Entgegenhaltungen:
- WO-A1-01/28682
- WO-A1-2004/011147
- WO-A2-2007/064404
- US-B1- 6 736 370
- CHAKRABORTY I ET AL: "MEMS micro-valve for space applications", SENSORS AND ACTUATORS A, ELSEVIER SEQUOIA S.A., LAUSANNE, CH, Bd. 83, Nr. 1-3, 1. Mai 2000 (2000-05-01), Seiten 188-193, XP004198313, ISSN: 0924-4247, DOI: 10.1016/S0924-4247(99)00382-9

## Beschreibung

Die vorliegenden Erfindung betrifft eine Vorrichtung zur Herstellung einer fluidischen Verbindung.

Die vorliegende Erfindung befasst sich insbesondere mit mikrofluidischen Systemen, insbesondere zur Diagnostik bei mikrofluidischen Proben, besonders bevorzugt bei sogenannten Point-Of-Care-Systemen (POC-System). Insbesondere befasst sich die vorliegende Erfindung mit vorzugsweise miniaturisierten fluidischen Systemen, im Folgenden Assays genannt, oder Immuno-Assays, also der Untersuchung von Proben unter Einsatz von Antikörpern. Die Erfindung kann sich aber auch auf sonstige, insbesondere miniaturisierte, fluidische Systeme beziehen. Besonders bevorzugt betrifft die vorliegende Erfindung sogenannte Cartridge-Konzepte, also kleine, insbesondere kartenförmige Vorrichtungen, zur Manipulation und/oder Untersuchung einer insbesondere flüssigen Probe bzw. der Realisierung von Assays oder Immuno-Assays.

Die DE 10 2007 035 721 A1 betrifft ein Mikroventil mit einer ersten und zweiten Ventilkammer, die miteinander verbindbar sind. Eine die Ventilkammern verschließende Membran wird durch Fluiddruck in einer der Kammern angehoben, wobei sich ein durchgehender Kanal zwischen den Ventilkammern bildet. Nachteilig ist jedoch, dass eine Verbindung zwischen den beiden Ventilkammern nur bei einer ausreichenden Druckdifferenz hergestellt werden kann. Eine Freisetzung eines in einer der Kammern befindlichen Reagenz zumindest im Wesentlichen ohne Druckdifferenz, durch eine äußere bzw. von zu verbindenden Einrichtungen unabhängige oder unabhängig steuerbare Krafteinwirkung und/oder durch mikrofluidischen Transport, insbesondere mit Kapillarkräften, ist folglich nicht möglich.

Die WO 2007/064404 A2 offenbart ein mikrofiuidisches System mit einem Substrat, zwei benachbarten Kavitäten und einer Membran, wobei die Membran vom Substrat anhebbar ist, um die Kavitäten fluidisch miteinander zu verbinden.

Chakraborty et al. offenbaren in der Veröffentlichung "MEMS micro-valve for space applications", Sensors and Actuators 83 (2000), 188-193, ein mikrofluidisches System mit einem Ventilsitz, einer Membran und zwei benachbarten Kavitäten, wobei die Membran mittels eines Aktors betätigbar ist, um die Kavitäten fluidisch miteinander zu verbinden.

Die WO 01/28682 A1 offenbart ein mikrofluidisches System mit einem Substrat, zwei benachbarten Kavitäten und einer flexiblen Membran, wobei die Membran mit ferromagnetischem Material oder Permanentmagneten versehen ist, um die Membran mittels eines Magneten zu betätigen und die Kavitäten fluidisch miteinander zu verbinden.

Der vorliegende Erfindung liegt die Aufgabe zugrunde, eine Vorrichtung zur Herstellung einer fluidischen Verbindung anzugeben, wobei eine Verbindung auch ohne Druckdifferenz und/oder durch äußere Krafteinwirkung hergestellt werden kann.

Die obige Aufgabe wird durch eine Vorrichtung gemäß Anspruch 1 gelöst. Vorteilhafte Weiterbildungen sind Gegenstand der Unteransprüche.

Die vorliegenden Erfindung befasst sich insbesondere mit der Freisetzung eines Reagenz oder sonstigen, insbesondere flüssigen oder fluidisch aktiven Stoffen, insbesondere in einem mikrofluidischen System. Unter "mikrofluidisch" sind erfindungsgemäß Volumina von vorzugsweise weniger als 10 ml, besonders bevorzugt weniger als 1 ml, und/oder Kanal-, Kavität- oder Flüssigkeitsquerschnitte (maximaler und/oder hydraulischer Durchmesser) von weniger als 5 mm, vorzugsweise weniger als 2 mm, besonders bevorzugt von weniger als 500 µm zu verstehen.

Gemäß einem ersten Aspekt der vorliegenden Erfindung weist eine Vorrichtung, insbesondere ein mikrofluidisches System, ein Substrat und zwei Kavitäten in räumlicher Nähe zueinander auf. Die Kavitäten sind durch eine gemeinsame Abdeckung gegeneinander abgedichtet. Weiter weist die Vorrichtung einen vorzugsweise stiftförmigen Körper auf, der aus einer Ausgangsposition in Richtung der Abdeckung unter Bildung einer fluidischen Verbindung der Kavitäten untereinander bewegbar ist.

Es wird insbesondere ein (mikrofluidisches) Ventil gebildet, das durch Bewegung des Körpers in eine Öffnungsposition geöffnet und vorzugsweise auch wieder verschlossen werden kann, insbesondere durch Bewegung des Körpers zurück in die Ausgangsposition. In vorteilhafter Weise ist hierdurch die Herstellung der fluidischen Verbindung ohne Druckdifferenz möglich und eine Freisetzung eines in einem der Kavitäten befindlichen Reagenz kann gesteuert werden.

Besonders bevorzugt ist der Körper in dem Substrat angeordnet, vorzugsweise wobei der Körpers auf einen Bereich der Abdeckung wirken kann, der beide Kavitäten abdeckt. Die Abdeckung kann durch den Körper verformt werden, vorzugsweise durch Aufwölben, durch Dehnung und/oder unter partieller Ablösung der Abdeckung von dem Substrat, insbesondere in dem Bereich. Vorzugsweise wird der Körper aus dem Substrat gegen die Abdeckung geschoben, wobei die Abdeckung bereichsweise ähnlich einem Zelt aufgespannt oder angehoben werden kann. Der von dem Substrat abgelöste Bereich der Abdeckung kann einen, insbesondere kegelstumpfförmigen und/oder mikrofluidisch aktiven, Hohlraum zwischen der Abdeckung und dem Substrat bilden, der insbesondere die fluidische Verbindung der Kavitäten bildet. Vorzugsweise sind die Kavitäten in dem Substrat angeordnet oder gebildet und können ganz oder teilweise durch die Abdeckung verschlossen sein. Die Kavitäten bilden mit der Abdeckung vorzugsweise mikrofluidisch aktive Kanäle, also insbesondere Kanäle mit einem Querschnitt, durch den ein Kapillartransport ermöglicht oder begünstigt wird. Die Abdeckung ist vorzugsweise auf dem Substrat angeordnet, insbesondere auf das Substrat aufgeklebt und/oder aufgeschweißt und kann eine Folie, Platte oder dgl. sein oder aufweisen.

Bei der vorliegenden Erfindung sind die Kavitäten, insbesondere in der Ausgangsposition des Körpers, durch die insbesondere auf einem dazwischenliegenden Bereich des Substrats dichtend aufliegende oder anhaftende Abdeckung gegeneinander abgedichtet. Hierzu kann die Abdeckung zumindest in einem Bereich zwischen den Kavitäten dicht, form- und/oder kraftschlüssig auf dem Substrat aufliegen. Vorschlagsgemäß kann die Abdeckung zwischen den Kavitäten unter Bildung fluidischen Verbindung durch den Körper verformt und/oder bewegt werden, vorzugsweise manuell, unabhängig von dem Fluiddruck in den Kavitäten, durch Einwirken einer äußeren, mechanischen und/oder manuellen Kraft, und/oder durch Aktivierung einer von den Kavitäten unabhängigen Einrichtung, die auch hydraulisch oder pneumatisch arbeiten kann.

Der Körper ist unter Bildung einer fluidischen Verbindung der Kavitäten untereinander gegen die Abdeckung verschiebbar. Es wird ein vorzugsweise stiftförmiger Körper verwendet, um die Fluidverbindung, insbesondere unter Anheben der Abdeckung in einem Bereich zwischen den Kavitäten, herzustellen. Hierbei kann das Substrat eine Ausnehmung, insbesondere Durchbrechung, aufweisen, in der der Körper beweglich, insbesondere verschiebbar, angeordnet und/oder (gleitend) gelagert ist. Die der Abdeckung abgewandte Seite der Ausnehmung ist mit einer weiteren Abdeckung versehen, was einen Eintritt von Fremdstoffen und/oder einen Austritt von Substanzen verhindern kann.
Gemäß einem weiteren Aspekt der vorliegenden Erfindung kann mindestens eine der Kavitäten einen Lagerbereich, ein Reservoir oder dergleichen bilden oder mit einem solchen verbunden sein. Um ein in diesem Lagerbereich befindliches Reagenz freizusetzen, werden die Kavitäten vorzugsweise in der eingangs beschriebenen Weise miteinander fluidisch verbunden.

Zur Herstellung der fluidischen Verbindung wird bevorzugt ein Druck oder eine sonstige Kraft von außen auf den vorzugsweise stiftförmigen Körper ausgeübt, wobei der stiftförmige Körper die Abdeckung zwischen den Kavitäten anhebt, einen Hohlraum oder Zwischenraum zwischen Substrat und Abdeckung erzeugt oder ermöglicht (beispielsweise zusammen mit dem Druck einer anstehenden Flüssigkeit) und dieser Hohlraum als fluidische Verbindung der Kavitäten untereinander wirkt. Somit kann das in dem Lagerbereich befindliche Reagenz insbesondere fluidisch von einer in die andere Kavität gelangen, ohne dass ein Überdruck zum Öffnen eines Ventils in einer der Kavitäten notwendig ist. In vorteilhafter Weise kann also die Freisetzung eines Reagenz oder einer sonstigen Substanz zumindest im Wesentlichen unabhängig von den Kavitäten, von einem Druck in den Kavitäten und/oder durch eine von außen wirkende, insbesondere steuerbare Kraft ermöglicht werden.

Ein weiterer Aspekt betrifft ein Verfahren zur Herstellung einer fluidischen Verbindung in einer mikrofluidischen Anordnung, wobei zwei benachbarte Kavitäten mit einer gemeinsamen Abdeckung derart versehen werden, dass die Abdeckung die Kavitäten gegeneinander abdichtet, und wobei eine fluidische Verbindung der Kavitäten untereinander durch Verformung der Abdeckung zwischen den Kavitäten mit einem vorzugsweise stiftförmigen Körper hergestellt wird.
Weitere Aspekte, Merkmale, Eigenschaften und Vorteile der vorliegenden Erfindung ergeben sich aus den Ansprüchen und der nachfolgenden Beschreibung einer bevorzugten Ausführungsform anhand der Zeichnung. Es zeigt:
- Fig. 1: eine ausschnittsweise Draufsicht einer vorschlagsgemäßen Vorrichtung;
- Fig. 2: einen ersten Schnitt der Vorrichtung gemäß Fig. 1; und
- Fig. 3: einen zweiten Schnitt durch die Vorrichtung gemäß Fig. 1.

In den Figuren werden für gleiche oder ähnliche Bauteile und Komponenten die gleichen Bezugszeichen verwendet, wobei sich entsprechende oder ähnliche Vorteile und Eigenschaften ergeben, auch wenn eine wiederholte Beschreibung weggelassen ist.

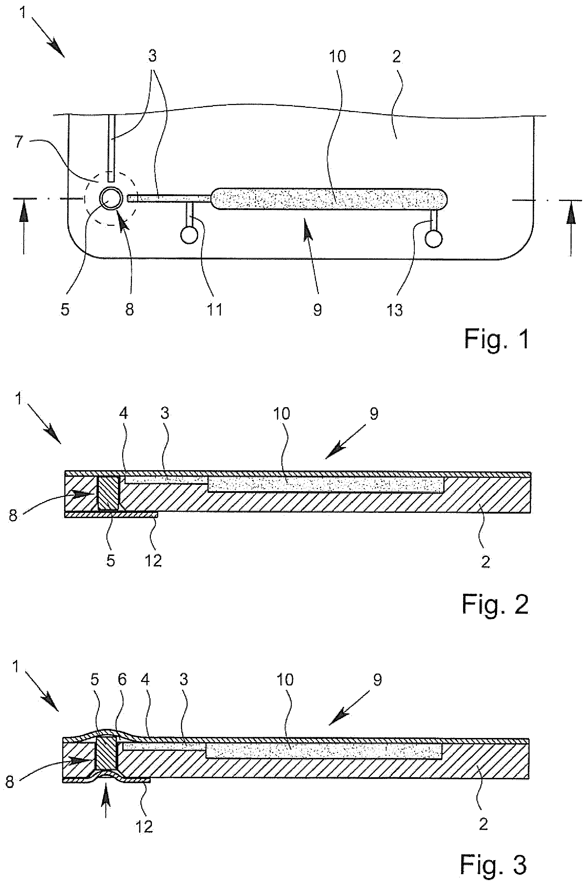

Fig. 1 zeigt in einer schematischen, ausschnittsweisen Draufsicht eine vorschlagsgemäße Vorrichtung 1, insbesondere ein mikrofluidisches System. Die Vorrichtung 1 weist zwei Kavitäten 3 auf, die in räumlicher Nähe bzw. benachbart zueinander angeordnet sind.

Fig. 2 zeigt einen Schnitt durch die Vorrichtung 1 entlang der in Fig. 1 angedeuteten Schnittlinie. Die Kavitäten 3 weisen eine gemeinsame Abdeckung 4 auf, die die Kavitäten 3 gegeneinander abdichtet. Diese Abdeckung 4 ist in Fig. 1 transparent bzw. aus darstellungstechnischen Gründen nicht abgebildet.

Die Vorrichtung 1 weist einen vorzugsweise stiftförmigen Körper 5 auf. Dieser kann vorschlagsgemäß aus einer Ausgangsposition, wie beispielhaft in Fig. 2 dargestellt, bei der die Kavitäten 3 gegeneinander abgedichtet sind, in Richtung der Abdeckung 4 in eine Öffnungsposition unter Bildung einer fluidischen Verbindung 6 der Kavitäten 3 bewegt werden. Vorzugsweise wird der Körper 5 hierzu in die in Fig. 3 mit dem Pfeil angedeutete Richtung bzw. in die in Fig. 3 gezeigte Öffnungsposition geschoben oder bewegt.

Besonders bevorzugt ist der Körper 5 in einer Ausnehmung 8, insbesondere Durchbrechung, des Substrats 2 angeordnet, vorzugsweise beweglich, insbesondere verschiebbar und/oder (gleitend) gelagert bzw. geführt. Die Ausnehmung 8 ist vorzugsweise zumindest im Wesentlichen komplementär zu dem Körper 5 ausgebildet. In vorteilhafter Weise ist es hierdurch möglich, dass ein Reagenz 10 durch die fluidische Verbindung 6 von einer in die andere Kavität 3 fließt, ohne das sich die Ausnehmung 8 mit Reagenz 10 (wesentlich oder in erheblicher Menge) füllt. Die Ausnehmung 8 verläuft vorzugsweise quer, insbesondere senkrecht, zur Haupterstreckungsebene der Abdeckung 4 und/oder des Substrats 2. Dies ermöglicht eine quer oder zumindest im Wesentlichen senkrecht gerichtete Bewegung des Körpers 5 gegen die Abdeckung 4, was die bevorzugte Ablösung der Abdeckung 4 von dem Substrat 2 zur Bildung der Verbindung 6 im Bereich der Kavitäten 3 begünstigt.

Der Körper 5 ist bevorzugt zylindrisch ausgebildet. Dies ermöglicht ein Einsetzen des Körpers 5 in die Ausnehmung 8 unabhängig von der Drehlage des Körpers 5 in Bezug auf seine Mittelachse senkrecht zu der Haupterstreckungsebene des Substrats 2 und/oder der Abdeckung 4.

Der Körper 5 kann gegen die Abdeckung 4 translatorisch und/oder durch eine in Richtung der Abdeckung 4 auf den Körper 5 wirkende Kraft bewegbar sein. Vorzugsweise bewirkt ein äußerer und/oder mechanischer Druck oder eine sonstige äußere Kraft auf die der Abdeckung 4 abgewandte Seite des Körpers 5 eine Verschiebung des Körper 5 aus seiner Ausgangsposition in seine Öffnungsposition zur Bildung der Verbindung 6, wie in Fig. 3 mit dem Pfeil angedeutet.

Der Körper 5 kann mit einer stumpfen oder flachen, der Abdeckung 4 zugewandten Stirnseite ausgebildet sein. Hierdurch kann der durch den Körper 5 auf die Abdeckung 4 zur Bildung der Verbindung 6 wirkende Druck auf eine Fläche verteilt werden, die zumindest im Wesentlichen der stirnseitigen Oberfläche des Körpers 5 entspricht. Somit kann dem Auftreten von Scherspannungen und einer Beeinträchtigung der Abdeckung 4 durch den Körper 5 vorgebeugt werden.

Der Körper 5 kann im Darstellungsbeispiel einen Durchmesser aufweisen, der kleiner als 5 mm, kleiner als 2 mm, vorzugsweise 1 mm oder geringer ist. Die Länge des Körpers 5 senkrecht zu der Haupterstreckungsebene der Abdeckung 4 und/oder des Substrats 2 ist vorzugsweise weniger als 10 mm oder 5 mm, insbesondere weniger als 2 mm. Der Bereich 7 kann beispielsweise einen Durchmesser aufweisen, der kleiner als 10 mm, vorzugsweise kleiner als 5 mm insbesondere 2 mm oder weniger ist.

Es ist besonders bevorzugt, dass die Abdeckung 4 durch den Körper 5 reversibel dehnbar ist. Es ist möglich, dass die gedehnte Abdeckung 4 eine Rückstellkraft auf den Körper 5 bewirkt, insbesondere wobei der Körper 5 durch die Rückstellkraft in seine Ausgangsposition (vgl. Fig. 2) bewegbar und/oder in einer Ausgangslage haltbar ist. Ein in Richtung der Abdeckung 4 ausgelenkter Körper 5 wird also bevorzugt durch die Spannung und/oder Elastizität der Abdeckung 4 in der in Fig. 2 gezeigten Position gehalten und/oder aus der in Fig. 3 gezeigten Position in die Position aus Fig. 2 zurückgedrückt.

In der Ausgangsposition bzw. Ausgangslage des Körpers 5 (Fig. 2) sind die Kavitäten 3 vorzugsweise gegeneinander abgedichtet. Wird der Körper 5 in Richtung der Abdeckung 4 ausgelenkt (Fig. 3), bildet sich die fluidische Verbindung 6. Bei Wegfall der den Körper 5 auslenkenden Kraft wird der Körper 5 vorzugsweise zurück in seine Ausgangslage (Fig. 2) geschoben, was insbesondere zu einer Rückbildung der Verbindung 6 und/oder einer Abdichtung der Kavitäten 3 untereinander führt. Alternativ kann durch Bewegung des Körpers 5 in die Öffnungsposition eine permanente Verbindung 6 erzeugt werden.

Ein Bereich 7 der Abdeckung 4 deckt besonders bevorzugt die Ausnehmung 8 und/oder Enden beider Kavitäten 3 ab. Der Bereich 7 kann insbesondere rund um die Ausnehmung 8 und/oder den Körper 5 angeordnet sein, wie in Fig. 1 durch die gestrichelte Linie angedeutet.

Die Abdeckung 4 ist vorzugsweise in dem Bereich 7 durch den Körper 5 verformbar, insbesondere durch Dehnung und/oder unter partieller Ablösung der Abdeckung 4 von dem Substrat 2, um die Kavitäten 3 untereinander fluidisch zu verbinden.

Der Bereich 7 der Abdeckung 4 ist bevorzugt aus dem gleichen Material wie der Rest der Abdeckung 4 gebildet. Insbesondere ist der Bereich 7 der Abdeckung 4 mit der Abdeckung 4 außerhalb des Bereichs 7 einstückig ausgebildet. Alternativ oder zusätzlich kann vorgesehen sein, dass die Abdeckung 4 im Bereich 7 ein anderes Material und/oder andere geometrische Eigenschaften aufweist. Insbesondere kann die Abdeckung 4 im Bereich 7 dünner oder dicker als die Abdeckung 4 in einem den Bereich 7 umgebenden Bereich ausgebildet sein.

Die Abdeckung 4 im Bereich 7 kann zumindest teilweise Wellen, Dehnungsfalten oder dergleichen aufweisen. Insbesondere kann in dem Bereich 7 der Abdeckung 4, vorzugsweise zwischen der Ausnehmung 8 und den Kavitäten 3, an oder durch die Abdeckung 4 ein Wulst, eine Dichtung oder dergleichen gebildet sein, insbesondere auf der dem Substrat 2 zugewandten Seite der Abdeckung 4.

Die Abdeckung 4 kann in dem Bereich 7 mit einem Klebstoff und/oder Dichtmittel beschichtet sein. Vorzugsweise kann die Abdeckung 4 in dem Bereich 7 eine Beschichtung aufweisen, insbesondere wobei diese sich von einer Beschichtung in anderen Bereichen der Abdeckung 4 unterscheidet. Der Bereich 7 kann eine Beschichtung aufweisen, die ein Verschweißen, Verkleben oder sonstiges permanentes Verbinden der Abdeckung 4 mit dem Substrat 2 in dem Bereich 7, insbesondere bei der Herstellung, verhindert und/oder eine spätere Ablösung der Abdeckung 4 im Bereich 7 von dem Substrat 2 begünstigt. Es ist also bevorzugt, dass die Abdeckung 4 zumindest im dem Bereich 7 derart ausgebildet ist, dass eine Abdichtung zwischen den Kavitäten 3 und/oder ein zerstörungsfreies Ablösen der Abdeckung 4 von dem Substrat 2 möglich ist. Die vorliegende Erfindung weist die Vorrichtung 1 auf der der Abdeckung 4 abgewandten Seite des Substrats 2 eine die Ausnehmung 8 überdeckende und/oder abdichtende weitere Abdeckung 12 auf. Die weitere Abdeckung 12 kann insbesondere auf das Substrat 2 aufgeklebt oder aufgeschweißt sein. Sie ist bevorzugt zur Weitergabe bzw. Übertragung einer Kraft, insbesondere von Druck und/oder durch Verformung, auf den Körper 5 ausgebildet. Wie in Fig. 3 mit dem Pfeil angedeutet, kann eine Kraft über die weitere Abdeckung 12 auf den Körper 5 wirken, vorzugsweise wobei der Körper 5 gegen die Abdeckung 4 geschoben wird. Hierdurch kann sich die Abdeckung 4 in dem Bereich 7 von dem Substrat 2 ablösen und/oder aufwölben, was zur Bildung der fluidischen Verbindung 6 führt. Die weitere Abdeckung 12 kann ein Eindringen von Fremdstoffen in die Ausnehmung 8 und/oder einen Austritt von Reagenz 10, insbesondere aus einer der Kavitäten 3, durch die Ausnehmung 8 auf der der Abdeckung 4 abgewandten Seite des Substrats 2 verhindern.

Die Ausnehmung 8 kann auf der der Abdeckung 4 abgewandten Seite des Substrats 2 einen konischen Eingangsbereich, insbesondere mit einem zur Öffnung hin zunehmenden Durchmesser, aufweisen. Hierdurch kann die Ausübung von mechanischem, insbesondere manuellem Druck, wie in Fig. 3 mit dem Pfeil angedeutet, erleichtert werden und/oder die Scherspannung an den eingangsseitigen Öffnungskanten der Ausnehmung 8 auf der der Abdeckung 4 abgewandten Seite des Substrats kann verringert werden. Zudem wird das Einsetzen des Körpers 5 in die Ausnehmung 8 erleichtert.

Die Abdeckung 4 und/oder die weitere Abdeckung 12 können eine Deckfolie sein oder aufweisen, vorzugsweise wobei die Abdeckung 4 und/oder 12 einlagig, mehrlagig, einseitig oder doppelseitig klebend, einschichtig oder mehrschichtig, mit mindestens einer niedrigschmelzenden Schicht oder Beschichtung, verformbar, elastisch und/oder flexibel ausgebildet ist.

Insbesondere kann die Abdeckung 4 und/oder 12 mehrschichtig ausgebildet sein, wobei im Bereich 7 auf Ausbildung einer der Schichten verzichtet wird und/oder eine zusätzliche Schicht vorgesehen ist, insbesondere wobei die zusätzliche Schicht elastisch rückfedernde Eigenschaften aufweist. Weiter kann die Abdeckung 4 und/oder 12 eine niedrigschmelzende Schicht oder Beschichtung aufweisen, insbesondere eine Schicht, die unter 200 °C, insbesondere unter 150 °C aufschmilzt. Diese niedrigschmelzende Schicht ist bevorzugt auf der dem Substrat 2 zugewandten Seite der Abdeckung 4 und/oder 12 angeordnet und kann zum Verschweißen und/oder thermischen Verkleben der Abdeckung 4 und/oder 12 mit dem Substrat 2 dienen. Alternativ oder zusätzlich kann eine ein- oder doppelseitig klebende Schicht, insbesondere auf der dem Substrat 2 zugewandten Seite der Abdeckung 4 und/oder 12, vorgesehen sein, was eine einfache und kostengünstige Montage der Abdeckung 4 und/oder der weiteren Abdeckung 12 zuträglich ist.

Gemäß einem Aspekt der vorliegenden Erfindung kann die Vorrichtung 1 einen Lagerbereich 9, insbesondere Reservoir, aufweisen, der mit einer durch die Abdeckung 4 abgedeckten Kavität 3 gebildet, und/oder mit der Kavität 3 kommunizierend verbunden ist. Der Lagerbereich 9 kann ein flüssiges und/oder festes Reagenz 10 aufweisen. Es ist besonders bevorzugt, dass das Reagenz 10 fluidisch transportabel ist, insbesondere also über Kapillarkräfte oder dergleichen befördert werden kann.

Ein Reagenz 10 im Sinne der vorliegenden Erfindung kann jede Substanz sein, die vorzugsweise mit einer anderen Substanz, insbesondere Probe und/oder Flüssigkeit, in Interaktion treten kann. Der Begriff "Reagenz" ist also vorzugsweise breit zu verstehen und umfasst auch Lösungsmittel, Waschsubstanzen oder dergleichen. Eine "Reaktion" kann also insbesondere auch eine rein physikalische oder physikalisch-chemische gegenseitige Beeinflussung zweier Substanzen sein, insbesondere auch ein Lösungsvorgang eines festen oder flüssigen Reagenz sein.

Der Lagerbereich 9 kann einen Zugang 11 und/oder eine Belüftung 13 aufweisen. Der Zugang 11 und/oder die Belüftung 13 können dazu verwendet werden, ein flüssiges und/oder festes Reagenz 10 einzufüllen und/oder den Lagerbereich 9 zu be- bzw. entlüften. Der Zugang 11 und/oder die Belüftung 13 sind vorzugsweise verschließbar, insbesondere durch Kleben, Aufkleben einer Folie, eines Klebestreifens und/oder durch Schweißen. Beim Füllen oder Entleeren des Lagerbereichs 9 mit einem Reagenz 10 ist es möglich, dass der Zugang 11 zur Belüftung und/oder die Belüftung 13 als Zugang verwendet wird.

Wie eingangs erläutert, wird der Körper 5 vorzugsweise von außen und/oder mechanisch, aus der Ausnehmung 8 in dem die Kavitäten 3 aufweisenden Substrat 2 derart gegen die Abdeckung 4 bewegt, dass die Abdeckung 4 zwischen den Kavitäten 3 von dem Substrat 2 abgehoben oder abgeschert und eine fluidische Verbindung 6 der Kavitäten 3 zueinander hergestellt wird. Es kann alternativ oder zusätzlich jedoch auch vorgesehen sein, dass der Körper 5 auf andere Weise in Bewegung gesetzt wird.

In einer Ausführungsform kann der Körper 5 ein magnetisches Material aufweisen und durch magnetische Abstoßung und/oder Anziehung aus dem Substrat 2 in Richtung der Abdeckung 4 bewegt werden. Auf, in oder in der Nähe des Substrats 2, vorzugsweise im Bereich 7 und/oder um die Ausnehmung 8 bzw. den Körper 5 herum kann eine elektrisch leitende Anordnung, insbesondere Spule, angeordnet sein, die vorzugsweise dazu verwendet werden kann, den Körper 5 magnetisch und/oder elektrostatisch zu bewegen. Alternativ oder zusätzlich kann es vorgesehen sein, dass der Körper 5 hydraulisch oder pneumatisch bewegt wird. Beispielsweise kann ein Gas- und/oder Flüssigkeitszugang zu der Aussparung 8 in dem Substrat 2 und/oder zu der dem Körper 5 abgewandten Seite der weiteren Abdeckung 12 vorgesehen sein, um einen hydraulischen oder pneumatischen Druck auf den Körper 5 auszuüben, insbesondere in die in Fig. 3 durch den Pfeil angedeutete Richtung. Es sind jedoch auch weitere, alternative Möglichkeiten zum Antrieb des Körpers 5 denkbar.

Gemäß einem weiteren, auch unabhängig realisierbaren Aspekt der vorliegenden Erfindung kann die vorschlagsgemäße Vorrichtung auch als oder zur Bildung einer, insbesondere mikrofluidischen, Pumpe verwendet werden. Hierzu ist es bevorzugt, dass die vorschlagsgemäße Vorrichtung mit einem Rückschlagventil oder sonstigen einen Materialfluss in eine Richtung hemmenden Einrichtung, insbesondere wie in der DE 10 2007 035 721 A1 offenbart, kombiniert wird.

Durch Verschieben des Körpers 5 in seine Öffnungsposition (Fig. 3) wird unter Ausbildung eines Hohlraums die fluidische Verbindung 6 erzeugt. Hierbei kann durch das zusätzliche Volumen ein Unterdruck erzeugt werden, was dazu führt, dass Reagenz 10 bei Erzeugung der fluidischen Verbindung 6 angesogen wird. Bei Bewegung des Körpers 5 in seine Ausgangsposition (Fig. 2) wird das Volumen des durch die fluidische Verbindung 6 erzeugten Hohlraums reduziert und in der fluidischen Verbindung 6 befindliches Reagenz 10 aus dem Bereich 7 herausgedrückt. Wird ein Rückfließen des Reagenz 10 beispielsweise durch ein Ventil in eine Richtung verhindert, wird das Reagenz 10 aus der fluidischen Verbindung 6 in die entgegengesetzte Richtung transportiert.

Durch mehrfaches Betätigen bzw. Bewegen des Körpers 5 zwischen Ausgangsposition und Öffnungsposition kann die vorschlagsgemäße Vorrichtung 1 also zum Pumpen verwendet werden.

### Bezugszeichenliste

- 1: Vorrichtung
- 2: Substrat
- 3: Kavität
- 4: Abdeckung
- 5: Körper
- 6: Verbindung
- 7: Bereich
- 8: Ausnehmung
- 9: Lagerbereich
- 10: Reagenz
- 11: Zugang
- 12: weitere Abdeckung
- 13: Belüftung

## Patentansprüche

1. Vorrichtung (1), insbesondere mikrofluidisches System, mit einem Substrat (2) und mit zwei benachbarten Kavitäten (3), die durch eine gemeinsame Abdeckung (4) gegeneinander abgedichtet sind, und mit einem vorzugsweise stiftförmigen Körper (5), der aus einer Ausgangsposition in Richtung der Abdeckung (4) unter Bildung einer fluidischen Verbindung (6) der Kavitäten (3) bewegbar ist, wobei das Substrat (2) eine Ausnehmung (8) aufweist, in der der Körper (5) verschiebbar angeordnet geführt ist, **dadurch gekennzeichnet, dass** die Vorrichtung (1) auf der der Abdeckung (4) abgewandten Seite des Substrats (2) eine die Ausnehmung (8) überdeckende und abdichtende weitere Abdeckung (12) aufweist, wobei die weitere Abdeckung (12) zur Weitergabe einer Kraft, insbesondere von Druck, auf den Körper (5) elastisch oder irreversibel verformbar ausgebildet ist.

2. Vorrichtung nach Anspruch 1, **dadurch gekennzeichnet, dass** die Kavitäten (3) in dem Substrat (2) angeordnet oder gebildet sind und von der Abdeckung (4) abgedeckt sind.

3. Vorrichtung nach einem der Ansprüche 1 oder 2, **dadurch gekennzeichnet, dass** die Abdeckung (4) zumindest in einem Bereich zwischen den Kavitäten (3) vorzugsweise flüssigkeitsdicht auf dem Substrat (2) aufliegt oder haftet.

4. Vorrichtung nach einem der voranstehenden Ansprüche, **dadurch gekennzeichnet, dass** die Abdeckung (4) folienartig ausgebildet oder eine Deckfolie ist.

5. Vorrichtung nach einem der voranstehenden Ansprüche, **dadurch gekennzeichnet, dass** der Körper (5) mit einer stumpfen, flachen oder abgerundeten der Abdeckung (4) zugewandten Stirnseite versehen ist.

6. Vorrichtung nach einem der voranstehenden Ansprüche, **dadurch gekennzeichnet, dass** durch den Körper (5) die Abdeckung (4) verformbar ist, insbesondere durch Dehnung und/oder unter partielle Auswölbung oder Ablösung der Abdeckung (4) von dem Substrat (2), um zumindest im Wesentlichen zwischen dem Substrat (2) und der Abdeckung (4) die fluidische Verbindung (6) der Kavitäten (3) zu bilden.

7. Vorrichtung nach einem der voranstehenden Ansprüche, **dadurch gekennzeichnet, dass** der Körper (5) gegen die Abdeckung (4) translatorisch und/oder durch eine in Richtung der Abdeckung (4) auf den Körper (5) wirkende Kraft bewegbar ist, insbesondere durch vorzugsweise äußeren Druck auf die der Abdeckung (4) abgewandte Seite des Körpers (5).

8. Vorrichtung nach einem der voranstehenden Ansprüche, **dadurch gekennzeichnet, dass** die Abdeckung (4) durch den Körper (5) reversibel dehnbar ist, vorzugsweise wobei die gedehnte Abdeckung (4) eine Rückstellkraft auf den Körper (4) bewirkt, insbesondere wobei der Körper (5) durch die Rückstellkraft in seine Ausgangsposition bewegbar und/oder in seiner Ausgangsposition haltbar ist.

9. Vorrichtung nach Anspruch1, **dadurch gekennzeichnet, dass** die Ausnehmung (8) und/oder Bewegungsrichtung des Körpers (5) quer, insbesondere senkrecht, zur Haupterstreckungsebene der Abdeckung (4) und/oder des Substrats (2) verläuft.

10. Vorrichtung nach einem der voranstehenden Ansprüche, **dadurch gekennzeichnet, dass** ein Lagerbereich (9) mit einer durch die Abdeckung (4) abgedeckten Kavität (3) gebildet, und/oder dass der Lagerbereich (9) mit einer der Kavitäten (3) kommunizierend verbunden ist, vorzugsweise wobei der Lagerbereich (9) ein flüssiges und/ader festes Reagenz (10) aufweist.

11. Vorrichtung nach Anspruch 10, **dadurch gekennzeichnet, dass** der Lagerbereich (9) einen verschließbaren Zugang (11) und/oder eine verschließbar Belüftung (13) in der Abdeckung (4) aufweist, vorzugsweise wobei durch den Zugang (11) und/oder die Belüftung (13) ein flüssiges und/oder festes Reagenz einfügbar ist, und/oder der Zugang (11) und/oder die Belüftung (13) durch Kleben, insbesondere durch Aufkleben einer Folie oder eines Klebestreifens, und/oder durch Schweißen verschließbar ist.

## Claims

1. Device (1), in particular microfluidic system, having a substrate (2) and two adjacent cavities (3), which are sealed off from each other by a common cover (4), and having a preferably pin-shaped body (5) that is movable out of a starting position in the direction of the cover (4) for forming a fluidic connection (6) of the cavities (3), wherein the substrate (2) has a recess (8) in which the body (5) is arranged movably guided, **characterized in that** the device (1) comprises, on the side of the substrate (2) remote from the cover (4), an additional cover (12) that covers and seals off the recess (8), wherein the additional cover (12) is designed to be elastically or irreversibly deformable in order to transmit a force, in particular pressure, to the body (5).

2. Device according to claim 1, **characterised in that** the cavities (3) are arranged or formed in the substrate (2) and are covered by the cover (4).

3. Device according to claim 1 or 2, **characterised in that** the cover (4) rests on or adheres, preferably liquid-tight, to the substrate (2) at least in a region between the cavities (3).

4. Device according to any one of the preceding claims, **characterised in that** the cover (4) is of film-like configuration or is a covering film.

5. Device according to any one of the preceding claims, **characterised in that** the body (5) is provided with a blunt, flat or rounded front end facing the cover (4).

6. Device according to any one of the preceding claims, **characterised in that** the cover (4) is deformable by the body (5), in particular by expansion and/or with partial bulging or detachment of the cover (4) from the substrate (2), in order to at least substantially form the fluidic connection (6) of the cavities (3) between the substrate (2) and the cover (4).

7. Device according to any one of the preceding claims, **characterised in that** the body (5) is movable towards the cover (4) by a translational movement and/or by a force acting on the body (5) in the direction of the cover (4), in particular by preferably external pressure on the side of the body remote from the cover (4).

8. Device according to any one of the preceding claims, **characterised in that** the cover (4) is reversibly expandable by the body (5), in particular wherein the expanded cover (4) exerts a restoring force on the body (4), in particular wherein the body (5) can be moved by the restoring force into its starting position and/or held in its starting position.

9. Device according to claim 1, **characterised in that** the recess (8) and/or direction of movement of the body (5) extends transversely, in particular perpendicularly, with respect to the main directional plane of the cover (4) and/or substrate (2).

10. Device according to any one of the preceding claims, **characterised in that** a storage region (9) is formed with a cavity (3) covered by the cover (4), and/or **in that** the storage region (9) is connected to one of the cavities (3) so as to communicate therewith, preferably wherein the storage region (9) contains a liquid and/or solid reagent (10).

11. Device according to claim 10, **characterised in that** the storage region (9) has a closable access point (11) and/or a closable vent (13) in the cover (4), preferably wherein a liquid and/or solid reagent (10) can be added through the access point (11) and/or the vent (13), and/or the access point (11) and/or the vent (13) is closable by adhesive bonding, in particular by adhesion of a film or an adhesive strip, and/or by welding.

## Revendications

1. Dispositif (1), en particulier système microfluidique, comprenant un substrat (2) et comprenant deux cavités (3) adjacentes, qui sont étanchéifiées l'une par rapport à l'autre par un recouvrement (4) commun, et comprenant un corps (5) de préférence en forme de tige, qui peut être déplacé depuis une position de départ en direction du recouvrement (4) pour formation une liaison fluidique (6) des cavités (3), dans lequel le substrat (2) présente un évidement (8), dans lequel le corps (5) est guidé en étant disposé de manière à pouvoir coulisser, **caractérisé en ce que** le dispositif (1) présente sur le côté opposé au recouvrement (4) du substrat (2) un autre recouvrement (12) recouvrant et étanchéifiant l'évidement (8), dans lequel l'autre recouvrement (12) est réalisé de manière élastiquement déformable ou de manière déformable irréversiblement afin de transférer une force, en particulier une pression, sur le corps (5).

2. Dispositif selon la revendication 1, **caractérisé en ce que** les cavités (3) sont disposées ou sont formées dans le substrat (2) et sont recouvertes par le recouvrement (4).

3. Dispositif selon l'une quelconque des revendications 1 ou 2, **caractérisé en ce que** le recouvrement (4) repose sur le substrat (2) ou y adhère au moins dans une zone entre les cavités (3) de préférence de manière étanche aux fluides.

4. Dispositif selon l'une quelconque des revendications précédentes, **caractérisé en ce que** le recouvrement (4) est réalisé à la manière d'un film ou est un film recouvrant.

5. Dispositif selon l'une quelconque des revendications précédentes, **caractérisé en ce que** le corps (5) est pourvu d'un côté frontal émoussé, plat ou arrondi tourné vers le recouvrement (4).

6. Dispositif selon l'une quelconque des revendications précédentes, **caractérisé en ce que** le recouvrement (4) peut être déformé par le corps (5), en particulier par l'allongement et/ou moyennant un bombement ou un décollement partiel du recouvrement (4) du substrat (2) afin de former au moins sensiblement entre le substrat (2) et le recouvrement (4) la liaison fluidique (6) entre les cavités (3).

7. Dispositif selon l'une quelconque des revendications précédentes, **caractérisé en ce que** le corps (5) peut être déplacé par translation contre le recouvrement (4) et/ou par une force agissant sur le corps (5) en direction du recouvrement (4), en particulier par une pression de préférence extérieure sur le côté, opposé au recouvrement (4), du corps (5).

8. Dispositif selon l'une quelconque des revendications précédentes, **caractérisé en ce que** le recouvrement (4) peut être allongé de manière réversible par le corps (5), dans lequel de préférence le recouvrement (4) allongé provoque une force de rappel sur le corps (4), en particulier dans lequel le corps (5) peut être déplacé par la force de rappel dans sa position de départ et/ou peut être maintenu dans sa position de départ.

9. Dispositif selon la revendication 1, **caractérisé en ce que** l'évidement (8) et/ou la direction de déplacement du corps (5) s'étendent de manière transversale, en particulier de manière perpendiculaire, par rapport au plan d'extension principal du recouvrement (4) et/ou du substrat (2).

10. Dispositif selon l'une quelconque des revendications précédentes, **caractérisé en ce qu'**une zone de palier (9) est formée avec une cavité (3) recouverte par le recouvrement (4), et/ou que la zone de palier (9) est reliée en communication avec une des cavités (3), de préférence dans lequel la zone de palier (9) présente un réactif (10) liquide et/ou solide.

11. Dispositif selon la revendication 10, **caractérisé en ce que** la zone de palier (9) présente un accès (11) pouvant être fermé et/ou une aération (13) pouvant être fermée dans le recouvrement (4), de préférence dans lequel un réactif liquide et/ou solide peut être transvasé par l'accès (11) et/ou par l'aération (13), et/ou l'accès (11) et/ou l'aération (13) peuvent être fermés par un collage, en particulier par l'application avec collage d'un film ou d'une bande adhésive, et/ou par un soudage.
